# EUROPÄISCHE PATENTSCHRIFT

(11) **EP 0 544 157 B1**
(45) Veröffentlichungstag und Bekanntmachung des Hinweises auf die Patenterteilung: **28.02.1996**
(21) Anmeldenummer: 92119402.3
(22) Anmeldetag: 13.11.1992
(51) Int. Cl.: C07C 31/20, C07C 29/60

(54) **Verfahren zur Herstellung von 1,2-Propylenglykol**
Process for the preparation of 1,2-propyleneglycol
Procédé de préparation du 1,2-propylèneglycol

(30) Priorität: 26.11.1991 DE 4138792
(43) Veröffentlichungstag der Anmeldung: 02.06.1993
(73) Patentinhaber: BASF Aktiengesellschaft, D-67063 Ludwigshafen (DE)
(72) Erfinder: Gehrer, Eugen, Dr., W-6700 Ludwigshafen (DE); Harder, Wolfgang, Dr., W-6940 Weinheim (DE)

(56) Entgegenhaltungen:
- EP-A- 0 007 101
- EP-A- 0 306 215
- EP-A- 0 405 956
- EP-A- 0 410 613
- EP-A- 0 468 320
- DE-C- 541 362
- DE-C- 899 190

## Beschreibung

Die vorliegende Erfindung betrifft ein Verfahren zur Herstellung von 1,2-Propylenglykol.

Die katalytische Hydrierung von Dihydroxyaceton führt bei Anwendung von Temperaturen von 20 bis 200°C zur Bildung von Glycerin (vgl. EP-A 405 956; EP-A 306 215; US-A 3 935 284; US-A 4024193; Sb. Tr. Vses. Zaochn. Politekh. Inst. 100, 7 (1976)-C.A. 90 5845 x) . Über die Bildung von 1,2-Propylenglykol wird in diesen Schriften nichts berichtet.

Die katalytische Hydrogenolyse von Glycerin zu 1,2-Propylenglykol ist ebenfalls bekannt (vgl. DE-PS 524 101; DE-PS 541 362; Z.Phys. Chem., Abt. A 159,337 (1932); J. Am. Chem. Soc. 54, 4678 (1932)). Diese Verfahren erfordern teilweise die Anwendung scharfer Reaktionsbedingungen, insbesondere die Anwendung hoher Reaktionstemperaturen, bei denen sich in hohem Maße Zersetzungsprodukte des Glycerins bilden, wodurch die Ausbeute erniedrigt wird. Teilweise sind diese Zersetzungsprodukte, insbesondere Ethylenglykol, destillativ schwierig vom gebildeten 1,2-Propylenglykol abzutrennen. Insgesamt ist die Ausbeute aller dieser Verfahren schlecht oder bestenfalls mäßig, sodaß sie für eine Anwendung im industriellen Maßstab nicht in Frage kommen.

1,2-Propylenglykol, eine wichtige Chemikalie, die z.B. in Frostschutzmitteln und Bremsflüssigkeiten Einsatz findet und als Diolkomponente bei der Herstellung von Polyurethanen und zur Herstellung von Polypropylenglykolen verwendet wird, wurde daher technisch bislang praktisch ausschließlich durch die Addition von Wasser an 1,2-Propylenoxid hergestellt (vgl. K. Weissermel; H.-J. Arpe: Industrielle Organische Chemie, 2. Auflage, S. 259 bis 260, Verlag Chemie, Weinheim 1978). Andere Verfahrenswege zur Herstellung von 1,2-Propylenglykol, wie die Herstellung aus Propen via 1,2-Diacetoxypropan und dessen anschließende Verseifung (US-A 3 715 388), erwiesen sich entweder als großtechnisch nicht durchführbar oder, wie die Herstellung von 1,2-Propylenglykol durch die Hydrogenolyse von Glycerin wegen der geschilderten Nachteile und Schwierigkeiten als wirtschaftlich uninteressant.

Da das Ausgangsmaterial Propylenoxid bislang industriell nicht über die Direktoxidation von Propen zugänglich ist, seine Herstellung nach dem Chlorhydrinverfahren mit Abwasser- und Nebenproduktproblemen behaftet ist, seine Herstellung nach einem der bekannten Cooxidationsverfahren zur - aus betriebswirtschaftlicher Sicht - unerwünschten Koppelproduktion mit anderen Produkten, wie Essigsäure, Methyl-tert.-butylether, Styrol oder Cyclohexanol, führt (vgl. K. Weissermel, H. -J. Arpe: Industrielle Organische Chemie, 2. Auflage, S. 251 bis 258, Verlag Chemie, Weinheim, 1978), lag der vorliegenden Erfindung die Aufgabe zugrunde, ein Verfahren zu finden, das die industriell wirtschaftliche Herstellung von 1,2-Propylenglykol, ausgehend von einer anderen Rohstoffbasis als Propylenoxid, ermöglicht.

Dementsprechend wurde ein Verfahren zur Herstellung von 1,2-Propylenglykol gefunden, das dadurch gekennzeichnet ist, daß man Dihydroxyaceton bei Temperaturen oberhalb 200°C mittels eines Hydrierkatalysators hydrogenolytisch in einer Stufe zu 1,2-Propylenglykol umsetzt.

Die einstufige katalytische Hydrogenolyse von Dihydroxyaceton läuft vermutlich über die bislang unbekannte Reaktionssequenz von Reaktionsschema (1) ab: 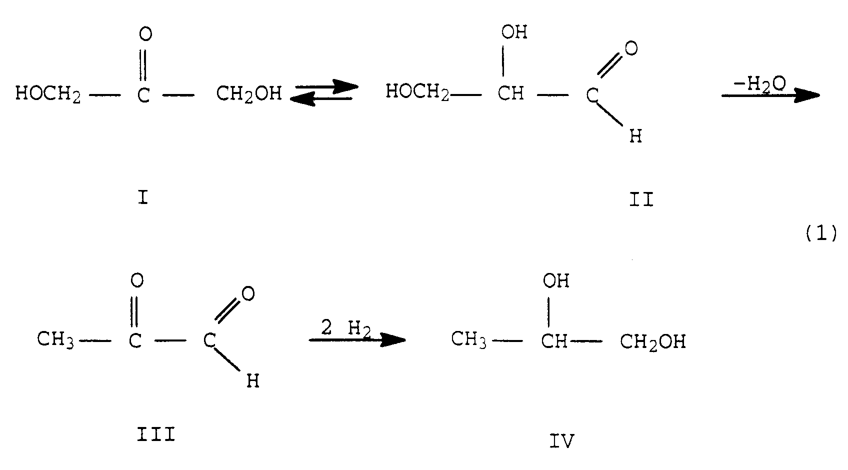
Dabei wird vermutlich aus dem mit Dihydroxyaceton I im Gleichgewicht stehenden Tautomer Glycerinaldehyd II unter den erfindungsgemäßen Reaktionsbedingungen Wasser abgespalten, wobei Methylglyoxal III entsteht, das anscheinend augenblicklich zum gewünschten 1,2-Propylenglykol IV hydriert wird. Zwar konnten bei den angewandten Reaktionsbedingungen die postulierten Zwischenprodukte II und III aufgrund ihrer offensichtlichen Instabilität unter den angewandten Reaktionsbedingungen in der Reaktionsmischung noch nicht nachgewiesen werden, dennoch erscheint dieser Reaktionsweg aufgrund der Ergebnisse von Vergleichsversuchen plausibel zu sein. So kann praktisch ausgeschlossen werden, daß die Herstellung von 1,2-Propylenglykol nach dem erfindungsgemäßen Verfahren über die Hydrogenolyse von intermediär gebildetem Glycerin abläuft. Unter identischen Reaktionsbedingungen wird Glycerin nur unvollständig und langsam und mit einer deutlich schlechteren Ausbeute und Selektivität zu 1,2-Propylenglykol umgesetzt. Unter der Hydrogenolyse des Dihydroxyacetons in einer Stufe wird somit erfindungsgemäß die hydrogenolytische Umwandlung von Dihydroxyaceton zu 1,2-Propylenglykol unter Bedingungen verstanden, bei denen die Hydrogenolyse des Dihydroxyacetons zu 1,2-Propylenglykol auf direktem Wege erfolgen kann, daß also die Stufe der Hydrogenolyse des isolierbaren Zwischenproduktes Glycerin nicht durchschritten werden muß.

Das erfindungsgemäße Verfahren wird bei Temperaturen oberhalb 200°C, bevorzugt bei Temperaturen von 210 bis 250°C und bei einem Wasserstoffdruck von im allgemeinen 1 bis 600 bar, vorzugsweise von 50 bis 200 bar und besonders bevorzugt bei einem Wasserstoffdruck von 80 bis 150 bar durchgeführt. Die Anwendung von hohen Temperaturen, insbesondere von Temperaturen oberhalb 200°C, ist für das erfindungsgemäße Verfahren kritisch, da bei niedrigen Temperaturen das Dihydroxyaceton hauptsächlich zu Glycerin hydriert wird, dessen Hydrogenolyse zu 1,2-Propylenglykol mit schlechteren Ausbeuten und Selektivitäten erfolgt als die einstufige Hydrogenolyse von Dihydroxyaceton zu 1,2-Propylenglykol. Bei Temperaturen oberhalb 250°C bilden sich vermehrt Spaltprodukte, wie Ethylenglykol, Propanol, Methan usw., weswegen die Anwendung solch hoher Temperaturen weniger bevorzugt ist, obgleich auch bei diesen hohen Temperaturen 1,2-Propylenglykol noch in beträchtlichen Mengen gebildet wird.

Das erfindungsgemäße Verfahren wird vorzugsweise heterogenkatalytisch durchgeführt. Dabei werden die Heterogenkatalysatoren vorteilhaft in einem Festbett angeordnet, über das die Reaktionsmischung in der Sumpf- oder vorzugsweise in der Rieselfahrweise geleitet wird. Bevorzugt wird das erfindungsgemäße Verfahren kontinuierlich betrieben, wobei zweckmäßigerweise Verweilzeiten des Reaktionsgutes am Katalysator von 0,5 bis 3 h, insbesondere von 0,75 bis 1,5 h eingestellt werden.

Die Hydrogenolyse des Dihydroxyacetons wird im allgemeinen in Lösung durchgeführt, wobei polare Lösungsmittel wie Wasser oder Alkohole, insbesondere C₁- bis C₂₀-Alkohole, beispielsweise Methanol, Ethanol, Propanol, Isopropanol, Butanole, 2-Ethylhexanol, Octanole, Nonanole, Decanole, Hexadecanol oder Octadecanol sowie mehrwertige Alkohole, wie Glycerin, bevorzugt werden. Besonders bevorzugt wird als Lösungsmittel 1,2-Propylenglykol verwendet, da sich hierdurch die destillative Aufarbeitung des Reaktionsproduktes besonders einfach gestaltet.

Zur Hydrogenolyse des Dihydroxyacetons können praktisch alle Arten von Hydrierkatalysatoren, insbesondere solche, die mindestens eines der Elemente der 6., 7., 8. und/oder 1. Nebengruppe des Periodensystems der Elemente enthalten, eingesetzt werden. Die Katalysatoren können als Vollkatalysatoren oder in Form von Trägerkatalysatoren eingesetzt werden, wobei als Trägermaterialien beispielsweise Aktivkohle, Bariumsulfat, Calciumcarbonat, Siliciumdioxid, Aluminiumoxide, Zirkoniumdioxid, Titandioxide, Kieselerden, gefällte amorphe Kieselsäure und/oder Silikate, z.B. Steatit, verwendet werden können. Besonders geeignet sind Katalysatoren, die eines oder mehrere der Elemente Chrom, Molybdän, Wolfram, Mangan, Rhenium, Ruthenium, Cobalt, Rhodium, Nickel, Eisen, Palladium, Platin, Kupfer und/oder Silber enthalten.

Im erfindungsgemäßen Verfahren können Hydrierkatalysatoren verwendet werden, die aus Metallen in aktivierter, feinverteilter Form mit großer Oberfläche bestehen, beispielsweise Raney-Nickel, Raney-Cobalt, Palladium- oder Platinschwamm.

Ebenso können die im erfindungsgemäßen Verfahren verwendbaren Katalysatoren die katalytisch aktiven Metalle auf einem inerten Trägermaterial abgeschieden enthalten. Als solche Katalysatoren wären z.B. die handelsüblichen Hydrierkatalysatoren Palladium auf Aktivkohle, Platin auf Aktivkohle, Ruthenium auf Aktivkohle, Rhodium auf Aktivkohle, Palladium auf Bariumsulfat, Palladium auf Bariumcarbonat, Palladium auf Calciumcarbonat, Platin auf Bariumsulfat, Platin auf Bariumcarbonat, Platin auf Aluminiumoxid, Platin auf Calciumcarbonat, Ruthenium auf Calciumcarbonat, Ruthenium auf Bariumcarbonat, Ruthenium auf Bariumsulfat, Rhenium auf Aktivkohle, usw. zu nennen.

Des weiteren können im erfindungsgemäßen Verfahren z.B. sogenannte Fällungskatalysatoren verwendet werden. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid-und/oder -carbonat-lösungen, als z.B. schwerlösliche Hydroxide, Oxidhydrate, basische Salze oder Carbonate ausfällt, die erhaltenen Niederschläge anschließend trocknet und diese durch Calcinierung bei im allgemeinen 300 bis 700°C, insbesondere 400 bis 600°C in die betreffenden Oxide, Mischoxide und/oder gemischtvalentigen Oxide umwandelt, welche durch eine Behandlung im Wasserstoffstrom bei in der Regel 200 bis 700°C, insbesondere bei 200 bis 400°C, zu den betreffenden Metallen und/oder oxidischen Verbindungen niederer Oxidationsstufe reduziert und in die eigentliche katalytisch aktive Form überführt werden. Bei der Herstellung von Fällungskatalysatoren, die ein Trägermaterial enthalten, kann die Fällung der katalytisch aktiven Komponenten in Gegenwart des betreffenden zugesetzten oder vorgefällten, festen Trägermaterials erfolgen, die katalytisch aktiven Komponenten können vorteilhaft aber auch gleichzeitig mit dem Trägermaterial aus den betreffenden Salzlösungen gefällt werden.

Beispielhaft seien als Katalysatoren, die im erfindungsgemäßen Verfahren vorteilhaft angewandt werden können, die Katalysatoren von DE-A 39 32 332, US-A-3 449 445, EP-A 44 444, EP-A 147 219, DE-A 39 04 083, DE-A 23 21 101, EP-A 415 202, DE-A 2 366 264 und EP-A 100 406 genannt. Als besonders bevorzugte Katalysatoren seien z.B. die Katalysatoren von EP-A 100 406 und DE-A 2 366 264 erwähnt.

Nach beendeter Umsetzung können die 1,2-Propylenglykol-haltigen Reaktionsausträge auf an sich herkömmliche Weise destillativ, bei Atmosphärendruck oder unter vermindertem Druck, aufgearbeitet werden.

Das erfindungsgemäße Ausgangsmaterial Dihydroxyaceton ist auf verschiedenerlei Weise erhältlich, beispielsweise auf fermentativem Wege, durch die mikrobielle Oxidation von Glycerin (vgl.: C. Rainbow; A.H. Rose: Biochemistry of Industrial Micro-organisms, S. 612-613, Academic Press, London 1963) oder vorzugsweise auf chemischem Wege, durch die Thiazoliumylid-katalysierte Selbstkondensation von Formaldehyd (vgl.: EP-A 410 613). Besonders bevorzugt wird das im erfindungsgemäßen Verfahren einzusetzende Dihydroxyaceton nach einem Verfahren hergestellt, bei dem die Selbstkondensation des Formaldehyds mit Hilfe des Thiazoliumylid-Katalysators in Abwesenheit acider oder basischer Verbindungen in aprotischen, polaren Lösungsmitteln durchgeführt wird, wobei als solche Lösungsmittel beispielsweise Ether, wie Tetrahydrofuran, Dioxan oder Trioxan, Ethylenglykoldimethylether, Ethylenglykoldiethylether, Diethylenglykoldimethylether, Nitrile, wie Acetonitril, Amide, wie N,N-Dimethylformamid, N,N-Diethylformamid, N,N-Dipropylformamid, N,N-Dibutylformamid, Hexamethylphosphorsäuretriamid, Lactame, wie N-Methylpyrrolidon, N-Ethylpyrrolidon, Sulfoxide und Sulfone, wie Dimethylsulfoxid und Sulfolan geeignet sind.

Als Katalysatoren für die Herstellung von Dihydroxyaceton aus Formaldehyd können Thiazoliumylide der Formel Va 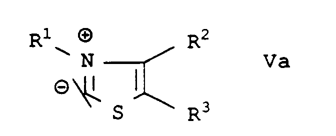
oder Benzothiazoliumylide der Formel Vb 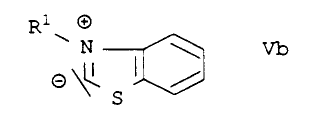
verwendet werden, in denen R¹ z.B. für eine C₁- bis C₃₀-Alkylgruppe, vorzugsweise eine C₂ bis C₂₀-Alkylgruppe oder eine Halogenalkylgruppe entsprechender Kohlenstoffzahl, die als Halogenatome vorzugsweise Fluor- oder Chloratome enthalten kann, eine an einen polymeren Träger gebundene C₁- bis C₂₅-Alkylengruppe, eine C₇- bis C₂₀-Aralkylgruppe, vorzugsweise eine C₇- bis C₁₂-Aralkylgruppe, insbesondere die Benzylgruppe oder eine Arylgruppe, wie die Phenyl- oder Naphthylgruppe, stehen kann und in denen die Reste R² und R³ gleich oder verschieden sein können und Wasserstoff, ein Halogenatom, eine C₁- bis C₁₀-Alkylgruppe, vorzugsweise eine C₁- bis C₄-Alkylgruppe, eine C₇- bis C₁₂-Aralkylgruppe, vorzugsweise die Benzylgruppe und/oder eine Phenyl- und/oder Naphthylgruppe bedeuten können. Die Reste R¹, R² und R³ können gewünschtenfalls noch unter den Reaktionsbedingungen inerte Substituenten tragen, wie Alkylgruppen, Alkoxygruppen, Halogenatome, C₂ bis C₁₀-Dialkylaminogruppen, C₁- bis C₁₀-Thioethergruppen, Nitrogruppen oder Nitrilogruppen.

Diese Thiazolium- bzw. Benzothiazoliumylid-Katalysatoren können aus den den entsprechenden Thiazoliumsalzen durch die Umsetzung mit einer Hilfsbase, beispielsweise einem tertiären Amin, erzeugt werden. Die Erzeugung der basen- und säure-freien Thiazoliumylid-Katalysatoren wird vorzugsweise nach zwei verschiedenen Methoden durchgeführt. So kann das Thiazoliumylid in einem unpolaren Lösungsmittel, wie Ethylenglykoldimethylether, erzeugt werden, indem das bei der Erzeugung des Thiazoliumylids durch die Umsetzung eines Thiazoliumsalzes mit einer Hilfsbase entstehende Salz der Hilsbase unlöslich ist, wonach das ausgefallene Salz der Hilfsbase von der so erhaltenen Katalysatorlösung mechanisch, beispielsweise durch Filtration oder Zentrifugation, abgetrennt werden kann. Die andere bevorzugte Methode zur Herstellung dieser basen- und säure-freien Thiazoliumylid-Katalysatoren besteht darin, daß man das Thiazoliumylid in einem polaren Lösungsmittel, wie Dimethylformamid, erzeugt und von dem bei der Erzeugung des Thiazoliumylids aus dem betreffenden Thiazoliumsalz mit einer Hilfsbase entstandenen löslichen Salz der Hilfsbase durch Kristallisation isoliert. Eine weitere Möglichkeit besteht in der in situ-Herstellung der Thiazoliumylid-Katalysatoren in der Reaktionsmischung zur Herstellung von Dihydroxyaceton durch die Umsetzung von N-Formyl-N-alkyl-o-mercaptoanilinen mit wasserentziehenden Mitteln, wie Orthoameisensäureestern.

Diese bevorzugte Art der Dihydroxyaceton-Herstellung liefert Dihydroxyaceton auf der Basis des preiswerten Ausgangsmaterials Formaldehyd in Ausbeuten bis zu 94 %. Diese bevorzugten Verfahren zur Herstellung von Dihydroxyaceton sind Gegenstand der deutschen Patentanmeldung Nr. P 4122669.0 und werden darin detailliert beschrieben.

Die Kombination dieses Verfahrens zur Herstellung von Dihydroxyaceton mit dem erfindungsgemäßen Verfahren zur Herstellung von 1,2-Propylenglykol aus Dihydroxyaceton ermöglicht nunmehr die kostengünstige Herstellung von 1,2-Propylenglykol ausgehend vom preiswerten Rohstoff Formaldehyd im industriellen Maßstab.

### Beispiele

In den Beispielen 1 bis 5 wurde ein Hydrierkatalysator gemäß EP-A 100 406 der folgenden Zusammensetzung verwendet:
66,0 Gew.-% Co, ber. als CoO
20,0 Gew.-% Cu, ber. als CuO
7,3 Gew.-% Mn, ber. als Mn₃O₄
3,6 Gew.-% Mo, ber. als MoO₃
3,0 Gew.-% P, ber. als H₃PO₄
Die Katalysatoren wurden vor ihrem Einsatz durch die Reduktion mit Wasserstoff bei Temperaturen von 200°C während einer Zeitdauer von 12 h aktiviert. Die Hydrogenolysen wurden alle in einem kontinuerlich betriebenen Rohrreaktor durchgeführt. Zur Bestimmung der Zusammensetzung der Hydrogenolyseprodukte wurden dem Reaktor, nachdem sich ein stationärer Betriebszustand eingestellt hatte, Proben entnommen und nach vorhergehender Silylierung gaschromatographisch untersucht.

### Beispiele 1 - 4

Die folgenden Beispiele zeigen, daß bei der Hydrierung von Dihydroxyaceton bei Temperaturen oberhalb 200°C eine dramatische Umkehr der Selektivität für Glycerin nach 1,2-Propylenglykol erfolgt.

Eine 20 gew.-%ige Lösung von Dihydroxyaceton in Wasser wurde bei 150 bar Wasserstoffdruck bei den in Tabelle 1 genannten Temperaturen mit Hilfe des oben erwähnten Katalysators umgesetzt. Der Katalysator wurde mit 50 ml Dihydroxyacetonlösung/h belastet (50 ml Katalysator in 50 ml Reaktor-Volumen).

Die Beispiele 1 bis 3 sind Vergleichsbeispiele.

Die Ergebnisse dieser Versuche sind in Tabelle 1 aufgeführt:

**Tabelle 1**

| Beispiel | Temperatur [°C] | Propylenglykol-Ausbeute [%] | Glycerin-Ausbeute [%] | Ethylenglykol-Ausbeute [%] |
|---|---|---|---|---|
| 1 | 120 | 2,6 | 96,7 | <0,1 |
| 2 | 150 | 11,2 | 89,3 | 0,15 |
| 3 | 180 | 15,6 | 74,0 | 0,47 |
| 4 | 210 | 89,2 | 6,3 | 3,7 |

### Beispiel 5 (Vergleichsbeispiel)

Dieses Beispiel zeigt, daß die Hydrogenolyse von Dihydroxyaceton zu 1,2-Propylenglykol über einen anderen Weg als über das Zwischenprodukt Glycerin verlaufen muß und daß die Hydrogenolyse von Glycerin zu 1,2-Propylenglykol unter identischen Bedingungen sehr viel langsamer und weniger selektiv verläuft als die des Dihydroxyacetons.

Beispiel 5 wurde wie Beispiel 4 ausgeführt, nur daß anstelle der Dihydroxyacetonlösung eine 20 gew.-%ige, wäßrige Glycerinlösung eingesetzt wurde.

Es entstanden 43,7 % 1,2-Propylenglykol, 16 % Propanol, 1,2 % Ethylenglykol sowie andere niedere Alkohole in geringeren Ausbeuten. 36,5 % des eingesetzten Glycerins wurden unter diesen Bedingungen nicht umgesetzt.

### Beispiel 6

Dieses Beispiel belegt, daß das erfindungsgemäße Verfahren zur einstufigen Hydrogenolyse von Dihydroxyaceton zu 1,2-Propylenglykol auch mit anderen Hydrierkatalysatoren durchgeführt werden kann.

Dihydroxyaceton wurde, wie in den Beispielen 1 bis 4 beschrieben bei den in Tabelle 2 angegebenen Temperaturen mittels eines Katalysators gemäß DE-A 2 366 264, der
55,6 Gew.-% Ni, ber. als NiO
15,3 Gew.-% Mg, ber. als MgO
25,6 Gew.-% Si, ber. als SiO₂ und
3,5 Gew.-% Mo, ber. als MoO₃
enthielt, zu 1,2-Propylenglykol umgesetzt.
Die Ergebnisse sind in Tabelle 2 aufgeführt.

| Temperatur [°C] | Propylenglykol-Ausbeute [%] | Glycerin-Ausbeute [%] | nicht umgesetztes Dihydroxyaceton |
|---|---|---|---|
| 180 | 39,7 | 52,0 | - |
| 200* | 64,3 | 23,2 | - |
| 220* | 67,0 | 0,4 | - |

| | | | |
|---|---|---|---|
| * Vergleichsversuch | | | |

## Patentansprüche

1. Verfahren zur Herstellung von 1,2-Propylenglycol, dadurch gekennzeichnet, daß man Dihydroxyaceton bei Temperaturen oberhalb 200°C mittels eines Hydrierkatalysators hydrogenolytisch in einer Stufe zu 1,2-Propylenglycol umsetzt.

2. Verfahren nach Anspruch 1, dadurch gekennzeichnet, daß man die Hydrogenolyse bei Temperaturen oberhalb 200°C bis 250°C durchführt.

3. Verfahren nach Anspruch 1, dadurch gekennzeichnet, daß man bei der Hydrogenolyse des Dihydroryacetons 1,2-Propylenglykol und/oder Glycerin und/oder Wasser als Lösungsmittel verwendet.

4. Verfahren nach Anspruch 1, dadurch gekennzeichnet, daß man Dihydroxyaceton einsetzt, das durch die Selbstkondensation von Formaldehyd in Gegenwart von Thiazoliumylid-Katalysatoren hergestellt worden ist.

5. Verfahren nach Anspruch 1, dadurch gekennzeichnet, daß man Katalysatoren verwendet, die mindestens eines der Elemente der 6., 7., 8. und/oder 1. Nebengruppe des Periodensystems der Elemente enthalten.

## Claims

1. A process for the preparation of 1,2-propylene glycol, which comprises reacting dihydroxyacetone by hydrogenolysis in one step using a hydrogenation catalyst at more than 200°C to give 1,2-propylene glycol.

2. The process as claimed in claim 1, wherein the hydrogenolysis is carried out at from above 200°C to 250°C.

3. The process as claimed in claim 1, wherein during the hydrogenolysis of the dihydroxyacetone, 1,2-propylene glycol and/or glycerol and/or water is used as solvent.

4. The process as claimed in claim 1, wherein the dihydroxyacetone employed has been prepared by the self-condensation of formaldehyde in the presence of thiazolium ylide catalysts.

5. The process as claimed in claim 1, wherein a catalyst is used which comprises at least one element from subgroup 6, 7, 8 and/or 1 of the Periodic Table of the Elements.

## Revendications

1. Procédé de préparation du 1,2-propylèneglycol, caractérisé en ce que l'on convertit la dihydroxyacétone à des températures supérieures à 200°C par voie hydrogénolytique et à l'aide d'un catalyseur d'hydrogénation en 1,2-propylèneglycol.

2. Procédé suivant la revendication 1, caractérisé en ce que l'on entreprend l'hydrogénolyse à des températures supérieures à 200°C et allant jusqu'à 250°C.

3. Procédé suivant la revendication 1, caractérisé en ce que l'on utilise le 1,2-propylèneglycol et/ou la glycérine et/ou l'eau comme solvant au cours de l'hydrogénolyse de la dihydroxyacétone.

4. Procédé suivant la revendication 1, caractérisé en ce que l'on utilise de la dihydroxyacétone qui a été préparée par l'autocondensation du formaldéhyde en présence de catalyseurs à base d'ylure de thiazolium.

5. Procédé suivant la revendication 1, caractérisé en ce que l'on utilise des catalyseurs qui contiennent au moins l'un des éléments du sixième, septième, huitième et/ou premier sous-groupes du système périodique des éléments.
